(19) Europäisches Patentamt
European Patent Office
Office européen des brevets

(11) **EP 1 817 547 B1**

(12) **EUROPÄISCHE PATENTSCHRIFT**

(45) Veröffentlichungstag und Bekanntmachung des Hinweises auf die Patenterteilung:
**18.04.2012 Patentblatt 2012/16**

(51) Int Cl.:
**G01C 21/16** (2006.01)   **A61B 19/00** (2006.01)

(21) Anmeldenummer: **05813615.1**

(22) Anmeldetag: **22.11.2005**

(86) Internationale Anmeldenummer:
**PCT/EP2005/012473**

(87) Internationale Veröffentlichungsnummer:
**WO 2006/058633 (08.06.2006 Gazette 2006/23)**

(54) **VERFAHREN UND EINE VORRICHTUNG ZUM NAVIGIEREN UND POSITIONIEREN EINES GEGENSTANDS RELATIV ZU EINEM PATIENTEN**

METHOD AND DEVICE FOR NAVIGATING AND POSITIONING AN OBJECT RELATIVE TO A PATIENT

PROCEDE ET DISPOSITIF DE NAVIGATION ET DE POSITIONNEMENT D'UN OBJET PAR RAPPORT A UN PATIENT

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **01.12.2004 DE 102004057933**

(43) Veröffentlichungstag der Anmeldung:
**15.08.2007 Patentblatt 2007/33**

(73) Patentinhaber: **Fraunhofer-Gesellschaft zur Förderung der angewandten Forschung e.V. 80686 München (DE)**

(72) Erfinder:
• **HAID, Markus 55268 Nieder-Olm (DE)**
• **SCHNEIDER, Urs 70374 Stuttgart (DE)**
• **VON LÜBTOW, Kai 70597 Stuttgart (DE)**

(74) Vertreter: **Friz, Oliver Dreiss Patentanwälte Postfach 10 37 62 70032 Stuttgart (DE)**

(56) Entgegenhaltungen:
**DE-A1- 4 205 869    DE-A1- 10 312 154
DE-C1- 4 225 112**

**Beschreibung**

Die Erfindung betrifft ein Verfahren und eine Vorrichtung zum Navigieren und Positionieren eines Gegenstands relativ zu einem Patienten während einer medizinischen Operation im Operationssaal. Beispielsweise kann es sich bei dem Prozess des Navigierens und Positionierens um die Anordnung einer Hüftprothese in einer exakt vorausberechneten Position relativ zum Oberschenkelknochen des Patienten handeln. Bislang wurde eine korrekte Positionierung durch Verwendung von Kameras im Operationssaal optisch überprüft. Hierbei waren auf dem zu navigierenden Gegenstand oder dem zu navigierenden Gerät optisch erkennbare Marken aufgebracht. Auch bei dem Patienten waren solche Marken vorgesehen. Die DE 4225112 beschreibt eine optische Positionierung. Ein derartiges System arbeitet zwar mit der erforderlichen Genauigkeit, es weist jedoch zahlreiche Nachteile auf. Ein hierfür erforderliches System mit mehreren Kameras und einer Bedieneinheit ist mit sehr hohen Kosten im Bereich mehrerer 10.000,-- € verbunden. Ein solches System arbeitet referenzbasiert, d. h. es ist prinzipiell nur stationär einsetzbar. Wenn das System an einem anderen Ort verwendet werden soll, so müssen die hierfür erforderlichen Kameras erneut an exakt vorherbestimmten Orten wieder aufgebaut werden. Des Weiteren erweist es sich als nachteilig, dass nur ein beschränkter Arbeitsbereich in der Größenordnung einer Abmessung von etwa 1 m zur Verfügung steht. Als besonders nachteilig erweist sich das Problem der Abschattung. Das System ist nur dann arbeitsfähig, wenn die hierfür verwandten Marken gleichzeitig von allen erforderlichen Kameras erfasst werden können. Wenn sich OP-Personal zwischen einer solchen Marke und einer Kamera aufhält oder sonstiges OP-Gerät dort positioniert wird, so ist das System nicht arbeitsfähig.

Der vorliegenden Erfindung liegt die Aufgabe zugrunde, ein Verfahren und eine Vorrichtung der eingangs beschriebenen Art dahingehend zu verbessern, dass die vorgenannten Nachteile nicht auftreten oder weitestgehend überwunden werden.

Diese Aufgabe wird durch ein Verfahren und eine Vorrichtung mit den Merkmalen der unabhängigen Ansprüche 1 und 3 gelöst.

Durch Anwendung des erfindungsgemäßen Verfahrens und der erfindungsgemäßen Vorrichtung lässt sich der Gegenstand, etwa ein Prothesenteil oder ein chirurgisches Gerät, in einer zuvor bestimmten Position am Patienten positionieren, oder anders ausgedrückt vermag das OP-Personal den betreffenden Gegenstand so bezüglich seiner Lage und Orientierung zu verändern, bis er die erwünschte vorbestimmte Position relativ zum Patienten oder relativ zu einem Bereich des Patienten erreicht hat. Die beanspruchte Vorrichtung ist mit weitaus geringeren Kosten verbunden als die Anwendung der eingangs beschriebenen Technik der optischen Erfassung. Das erfindungsgemäße System ist weitestgehend transportabel; es erfordert mit Ausnahme der an dem Gegenstand und dem Patienten befestigbaren Sensoreinrichtungen keine größeren Gerätschaften, die aufwendig an exakt vorbestimmten Positionen stationär montiert werden müssten. Die eine dreidimensionale Inertialsensorik aufweisenden Sensoreinrichtungen sind auch weitgehend miniaturisierbar, sie lassen sich in Volumina von wenigen Millimetern Abmessung realisieren. Diese Sensoreinrichtungen sind an einer vorbestimmten Stelle und in einer vorgegebenen Orientierung an dem besagten Gegenstand einerseits und an einer ebenso vorbestimmten Position am Patienten andererseits befestigbar und wieder lösbar. Die Sensoreinrichtungen weisen hierfür vorteilhafterweise eine vorzugsweise visuell erkennbare Orientierungshilfe auf, die eine korrekte Festlegung an dem Gegenstand bzw. an dem Patienten gestattet.

Es erweist sich auch als vorteilhaft, wenn die Sensoreinrichtungen Befestigungsmittel aufweisen zum lösbaren Festlegen der Sensoreinrichtung an dem Gegenstand und an dem Patienten. Hierbei kann es sich beispielsweise um klemmende oder klipsartig ausgebildete Befestigungsmittel handeln.

Um im Zuge der Durchführung des Verfahrens Positionsdaten aus den Messwerten der ersten Sensoreinrichtung und der zweiten Sensoreinrichtung miteinander vergleichen zu können, ist es lediglich erforderlich, dass zu Beginn der Positionierung eine Referenzierung der Sensoreinrichtungen durchgeführt wird, indem beide Sensoreinrichtungen an einem gemeinsamen Ort oder an zwei in vorbestimmter bekannter Orientierung zueinander liegenden Orten zur Ruhe gebracht werden. Hiervon ausgehend wird dann durch Verwendung der dreidimensionalen Sensorik eine Verlagerung der Sensoreinrichtungen ermittelt und weiterer Datenverarbeitung zugeführt.

Die erste und insbesondere auch die zweite Sensoreinrichtung weisen bevorzugtermaßen drei Beschleunigungssensoren auf, deren Signale zur Berechnung einer translatorischen Bewegung verwendbar sind, und zusätzlich drei Drehratensensoren, deren Messwerte zum Bestimmen der Orientierung im Raum verwendbar sind.

Zusätzlich zu den Drehratensensoren können in vorteilhafter Weise Magnetfeldsensoren zur Ermittlung der Orientierung des Gegenstands im Raum vorgesehen sein. Die Magnetfeldsensoren erfassen dabei Komponenten des Erdmagnetfelds und vermögen ihrerseits Informationen über die Orientierung der Sensoreinrichtung im Raum zu geben.

Solchenfalls erweist es sich als vorteilhaft, wenn Mittel zum Vergleichen der aus Messwerten der Magnetfeldsensoren ermittelten Orientierung im Raum mit der aus Messwerten der Drehratensensoren ermittelten Orientierung im Raum vorgesehen sind. Zusätzlich kann ein Beschleunigungssensor für die Messung der Erdbeschleunigung vorgesehen sein, welcher ebenfalls zur Ermittlung der Orientierung der betrachteten Sensoreinrichtung im Raum verwendet werden kann.

Es erweist sich des Weiteren als besonders vorteilhaft, wenn die Rechenmittel der erfindungsgemäßen Vorrichtung Mittel zur Ausführung eines an sich aus DE 103 12 154 A1 bekannten Quaternionen-Algorithmus aufweisen.

Des Weiteren erweist es sich als vorteilhaft, wenn die Rechenmittel Mittel zur Anwendung einer vor Beginn der Positionierung ermittelten und gespeicherten Ausgleichsmatrix aufweisen, welche Ausgleichsmatrix einer Abweichung der Orientierung der Achsen der drei Drehratensensoren von einer angenommenen Orientierung der Achsen zueinander Rechnung trägt und durch ihre Anwendung einen bei der Berechnung der Drehwinkel resultierenden Fehler kompensiert.

Ferner erweist es sich als vorteilhaft, wenn die Rechenmittel Mittel zur Ausführung eines Kalman-Filter-Algorithmus aufweisen.

Da Inertialsensoren auf Beschleunigungsvorgänge zurückgehende Messwerte liefern, werden diese Messwerte zur Bestimmung von Positionsdaten im Falle von Beschleunigungssensoren zweifach integriert und im Falle von Drehratensensoren einfach integriert. Im Zuge dieser Integrationsvorgänge werden Fehler nach und nach durch Integration aufaddiert. Insofern erweist es sich als vorteilhaft, dass das Verfahren und die hierfür verwandte Vorrichtung so ausgebildet ist, dass vor Beginn einer Datennahme und dann immer wieder, sobald ein Ruhen der Vorrichtung für eine bestimmte Zeitdauer festgestellt wird, ein Offset-Wert des Ausgangssignals der Drehratensensoren ermittelt wird und nachfolgend bis zur nächstfolgenden Ermittlung dieser Offset-Wert für die jeweiligen Drehratensensoren in Abzug gebracht wird, so dass er nicht in die Integration eingeht. Auf diese Weise wird sichergestellt, dass immer wieder ein neuer aktueller Offset-Wert ermittelt wird, so dass größtmögliche Genauigkeit erreicht wird.

Es wurde auch festgestellt, dass die konstruktionsbedingt nicht zu vermeidende Abweichung der Orientierung der Achsen der drei Drehratensensoren von einer angenommenen Orientierung sehr rasch bei der Berechnung der Drehwinkel zu Ungenauigkeiten führt. Dadurch, dass dieser Fehler durch Anwendung der für die betreffende Sensoreinrichtung zu bestimmenden Ausgleichsmatrix kompensiert wird, lässt sich eine den Anforderungen genügende Steigerung der Genauigkeit erreichen. Zur Ermittlung der Ausgleichsmatrix wird vor Beginn der Objektverfolgung eine hierfür verwandte Sensoreinrichtung in eine rotierende Bewegung um eine jeweilige Achse gebracht, und zwar unter Stillsetzung der beiden anderen Achsen. Anhand der hierbei erhaltenen Drehratensensorsignale wird die Ausgleichsmatrix errechnet und in einem Speicher der beanspruchten Vorrichtung hinterlegt. Für den rotatorischen Antrieb der Sensorvorrichtung kann ein Industrieroboter verwendet werden. Auf diese Weise kann die 3x3-Nichtorthogonalitätsmatrix durch Drehung um die einzelnen Raumachsen nacheinander aufgenommen werden.

$$\underline{\underline{N}} = \begin{pmatrix} N_{11} & N_{12} & N_{13} \\ N_{21} & N_{22} & N_{23} \\ N_{31} & N_{32} & N_{33} \end{pmatrix} \qquad\qquad (1)$$

Die Nebendiagonalelemente der Nichtorthogonalitätsmatrix in Gleichung wären bei einem idealen System 0. Die Ungenauigkeiten beruhen auf Fertigungsungenauigkeiten, die dazu führen, dass die Achsen der Drehratensensoren weder in einer vorbestimmten Orientierung zu einem Gehäuse der Sensoreinrichtung noch exakt orthogonal zueinander angeordnet sind.

Wenn vorausgehend davon die Rede war, dass ein Offset-Wert bestimmt wird, wenn ein Ruhen der Vorrichtung für eine bestimmte Zeitdauer festgestellt wird, so wird hierunter die Bestimmung eines Driftvektors verstanden, und zwar für drei vorzugsweise orthogonal zueinander orientierte Drehratensensoren und für drei vorzugsweise orthogonal zueinander orientierte Beschleunigungssensoren. Auf diese Weise kann eine Matrix D ermittelt werden, deren Zeilen die Offsets der einzelnen Sensoren sind. Sie werden vorzugsweise zu Beginn einer Objektverfolgung und dann immer wieder bei einer detektierten Ruhelage neu ermittelt und für die weitere Datenverarbeitung zugrunde gelegt.

$$\underline{\underline{D}} = \begin{pmatrix} D_1 \\ D_2 \\ D_3 \end{pmatrix} \qquad\qquad (2)$$

Die Genauigkeit der Bestimmung der Orientierung wird nach einer Ausführungsform der Erfindung auch dadurch erhöht, dass bei jeder Messdatennahme und Ermittlung der drei Drehwinkel ein Quaternionen-Algorithmus der nachfolgend definierten Art auf die drei Drehwinkel angewandt wird, um hieraus die aktuelle Orientierung des Objekts im Raum zu errechnen. Die hierdurch erzielte weitere Verbesserung beruht auf folgendem Umstand: Wenn man die durch einfache Integration bei jedem infinitesimalen Schritt der Abtastung, d. h. der Messdatennahme erhaltbaren infinitesimalen Drehwinkel um eine jeweilige Achse zur Bestimmung der Orientierungsänderung des Objekts so heranziehen würde, dass die Drehungen nacheinander um die jeweilige Achse durchgeführt würden, so würde hieraus ein Fehler resultieren. Dieser Fehler liegt darin begründet, dass die Messdaten der drei Sensoren zum gleichen Zeitpunkt genommen werden, weil eine Drehung in der Regel gleichzeitig um drei Achsen stattfindet und ermittelt wird. Wenn dann zur Ermittlung der Positionsveränderung die drei bestimmten Drehwinkel aber nacheinander als Drehungen um die jeweiligen Achsen berücksichtigt würden, so würde bei der Drehung um die zweite und die dritte Achse ein Fehler resultieren, da diese Achsen bereits im Zuge der ersten Drehung fehlerbehaftet in eine andere Orientierung gebracht wurden. Dem wird durch Anwendung des Quaternionen-Algorithmus auf die drei Drehwinkel begegnet. Auf diese Weise werden die drei Drehungen durch eine einzige Transformation ersetzt. Der Quaternionen-Algorithmus ist folgendermaßen definiert:

Die Quaternion ist in Gl.1. definiert:

$$(3) \qquad \underline{q} = q_0 + q_1 \cdot i + q_2 \cdot j + q_3 \cdot k$$

mit den Bedingungen Gl.4. bis Gl.8.

$$(4) \qquad q_{0..3} \in \mathfrak{R}$$

$$(5) \qquad i^2 = j^2 = k^2 = -1$$

$$(6) \qquad i \cdot j = -j \cdot i = k$$

$$(7) \qquad j \cdot k = -k \cdot j = i$$

$$(8) \qquad k \cdot i = -i \cdot k = j$$

Durch Zusammenfassen der komplexen Anteile zu einem Vektor v und mit $q_0$=w gelangt man zu der Schreibweise in Gl.9.

$$(9) \qquad \underline{q} \in (w \cdot \underline{v})^T$$

mit den Bedingungen Gl.10. und Gl.11.

(10)

$$w \in \mathfrak{R}$$

(11)

$$\underline{v} \in \mathfrak{R}^3$$

Für die Benutzung der Quaternionen gelten die Definitionen Gl. 12 bis Gl.17.
Konjugierte Quaternion

(12)

$$\underline{q}^k = \begin{pmatrix} w \\ -\underline{v} \end{pmatrix}$$

Betrag

(13)

$$\left| \underline{q} \right| = \sqrt{w^2 + \underline{v} \cdot \underline{v}}$$

Inversion

(14)

$$\underline{q}^{-1} = \frac{\underline{q}^k}{\left| \underline{q} \right|}$$

Multiplikation

(15)

$$\underline{q}_1 \cdot \underline{q}_2 = \begin{pmatrix} w_1 \\ \underline{v}_1 \end{pmatrix} \cdot \begin{pmatrix} w_2 \\ \underline{v}_2 \end{pmatrix} = \begin{pmatrix} w_1 \cdot w_2 + \underline{v}_1 \cdot \underline{v}_2 \\ w_1 \cdot \underline{v}_2 + w_2 \cdot \underline{v}_1 + \underline{v}_1 \times \underline{v}_2 \end{pmatrix}$$

Darstellung eines Vektors

(16)

$$\underline{q}_v = \begin{pmatrix} 0 \\ \underline{v} \end{pmatrix}$$

Darstellung eines Skalars

(17)

$$\underline{q}_w = \begin{pmatrix} w \\ \underline{0} \end{pmatrix}$$

Besondere Bedeutung für die inertiale Objektverfolgung erhält die Multiplikation. Diese stellt eine Rotation einer Quaternion dar. Dafür wird eine Rotationsquaternion Gl.18. eingeführt.

$$(18) \qquad \underline{q}_{rot} = \begin{pmatrix} \cos\left(\dfrac{|\phi|}{2}\right) \\ \sin\left(\dfrac{|\phi|}{2}\right) \cdot \dfrac{\phi}{|\phi|} \end{pmatrix}$$

Der Vektor $\phi$ besteht aus den einzelnen Drehungen um die Koordinatenachsen.

Eine Drehung eines Punkts oder Vektors kann nun wie folgt errechnet werden. Zunächst müssen die Koordinaten des Punkts oder der Vektor mittels Gleichung Gl.16 in eine Quaternion umgewandelt und anschließend die Multiplikation mit der Rotationsquaternion durchgeführt werden (Gl.18). Die Ergebnisquaternion enthält den rotierten Vektor in der gleichen Schreibweise. Unter der Voraussetzung, dass der Betrag einer Quaternion gleich eins ist, kann die invertierte Quaternion durch die konjugierte ersetzt werden (Gl.19).

$$(19) \qquad \underline{q}_{v'} = \underline{q}_{rot} \cdot \underline{q}_{v} \cdot \left(\underline{q}_{rot}\right)^{-1} = \underline{q}_{rot} \cdot \underline{q}_{v} \cdot \left(\underline{q}_{rot}\right)^{-k}$$

wegen Gl. 20.

$$(20) \qquad \left|\underline{q}_{rot}\right| = 1$$

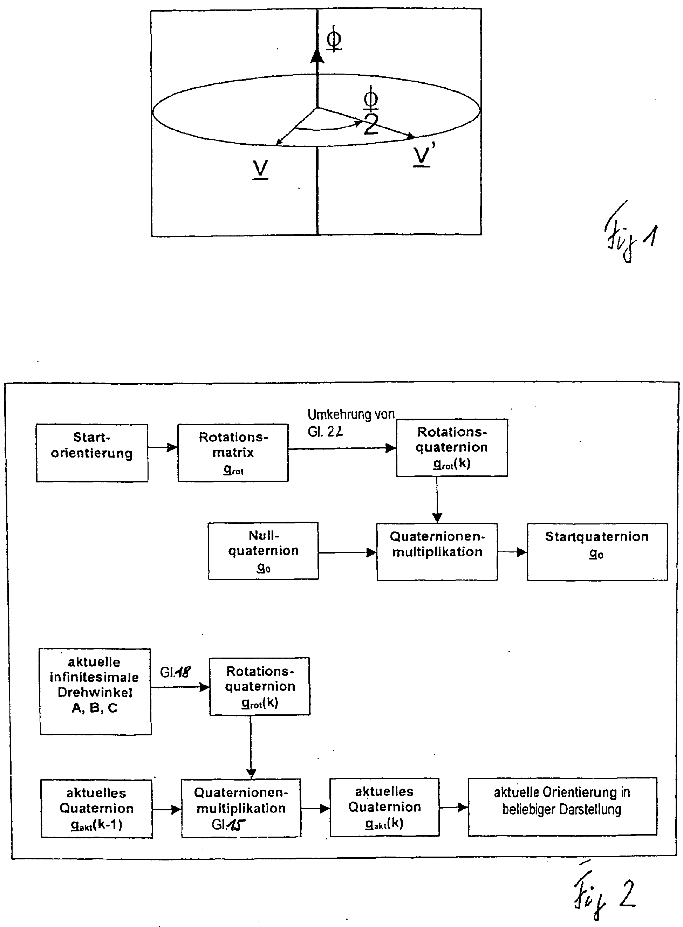

Wie kann man diese Operation verdeutlichen? Der vektor $\phi$ ist der Normalenvektor zu der Ebene, in der eine Drehung um den Winkel $\frac{1}{2}\phi$ ausgeführt wird. Der Winkel entspricht dem Betrag des Vektors $\phi$. Siehe Fig.1.

Aus Fig.1 ist ersichtlich, dass eine Rotation in jeder beliebigen Ebene und unter Angabe nur eines Winkels durchgeführt werden kann. Darin zeigen sich auch die besonderen Vorteile für dieses Verfahren. Weitere Vorteile sind die geringe Anzahl an notwendigen Parametern und trigonometrischer Funktionen, welche durch Näherungen für kleine Winkel völlig ersetzt werden könnten. Für die Rotation mit dem Vektor $\omega$ gilt die Differenzialgleichung 21:

$$(21) \qquad \frac{d}{dt}\underline{q}_{rot} = \frac{1}{2}\underline{q}_{rot} \cdot \begin{pmatrix} 0 \\ \underline{\omega} \end{pmatrix}$$

Die konkrete Umsetzung des Quaternionen Algorithmus ist in Figur 2 dargestellt und erfolgt folgendermaßen: Insgesamt erfolgt die gesamte Berechnung mit Hilfe von Einheitsvektoren. Ausgehend von der Startorientierung werden die Starteinheitsvektoren $E_x$, $E_y$ und $E_z$ ermittelt.

Aus den Einheitsvektoren wird mit Hilfe von Gleichung Gl. 22 die Rotationsmatrix berechnet.

$$(22) \quad R = \begin{bmatrix} (q_0)^2 + (q_1)^2 - (q_2)^2 - (q_3)^2 & 2 \cdot (q_1 \cdot q_2 - q_0 \cdot q_3) & 2 \cdot (q_1 \cdot q_3 - q_0 \cdot q_2) \\ 2 \cdot (q_1 \cdot q_2 - q_0 \cdot q_3) & (q_0)^2 - (q_1)^2 + (q_2)^2 - (q_3)^2 & 2 \cdot (q_2 \cdot q_3 - q_0 \cdot q_1) \\ 2 \cdot (q_1 \cdot q_3 - q_0 \cdot q_2) & 2 \cdot (q_2 \cdot q_3 - q_0 \cdot q_1) & (q_0)^2 - (q_1)^2 - (q_2)^2 + (q_3)^2 \end{bmatrix}$$

Ausgehend von einer Startorientierung des mit dem Objekt verbundenen Koordinatensystems, insbesondere ausgehend von sogenannten Einheitsstartvektoren, wird gemäß Gleichung 22 die Rotationsmatrix R, bei der es sich um eine 3x3-Matrix handelt, errechnet. Aus einer Umkehrung dieser Gleichung 22 lässt sich ein Rotationsquaternion $q_{rot}(k)$ erhalten. Mit Hilfe des Nullquaternions, welches sich aus den Nulleinheitsvektoren ergibt, wird durch eine Multiplikation mit dem Rotationsquaternion das Startquaternion berechnet. Bei der nächstfolgenden Abtastung, also bei der nächsten Messdatennahme und Integration zu aktuellen infinitesimalen Drehwinkeln A, B, C errechnet sich dann ein für diesen Schritt zu benutzendes Rotationsquaternion $q_{rot}(k)$. Das im vorherigen Schritt gebildete Quaternion $q_{akt}(k-1)$ wird dann mit diesem Rotationsquaternion $q_{rot}(k)$ gemäß Gleichung 13 multipliziert, um das aktuelle Quaternion des vorliegenden k-ten Schritts, nämlich $q_{akt}(k)$ zu erhalten. Aus diesem aktuellen Quaternion kann dann für den gerade durchgeführten Abtastschritt die aktuelle Orientierung des Objekts in beliebiger Darstellung angegeben werden.

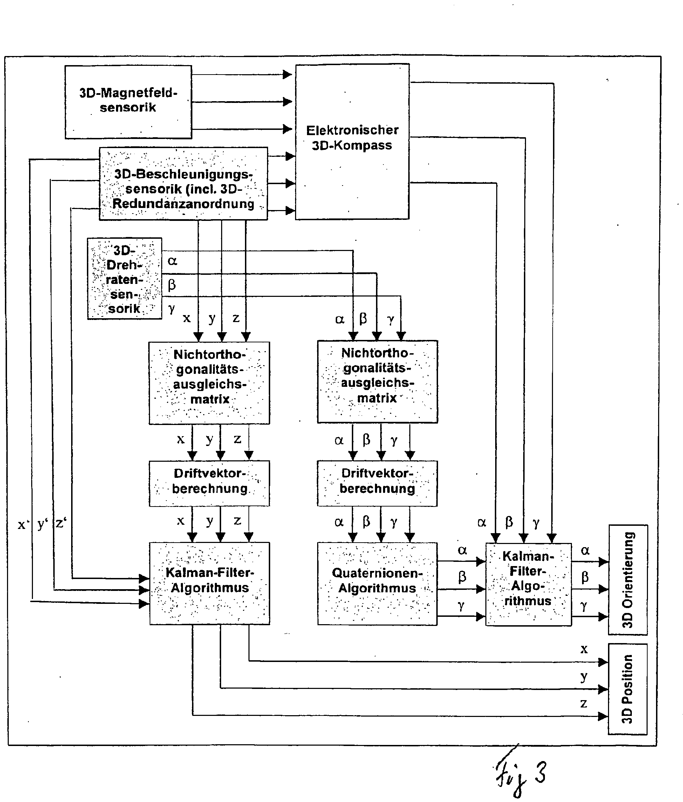

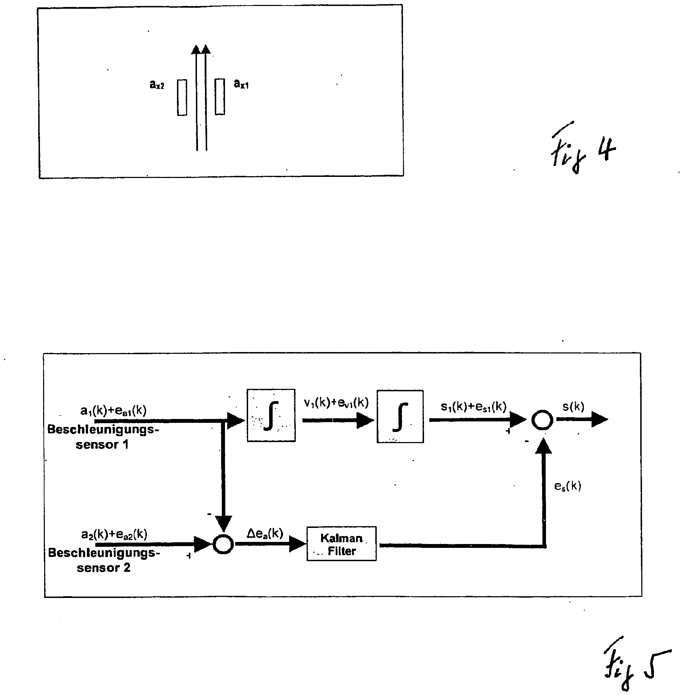

Es wurde vorausgehend schon darauf hingewiesen, dass ein Kalman-Filteralgorithmus angewandt werden kann, um die Genauigkeit bei der Ermittlung oder Errechnung von Positionsdaten zu erhöhen. Das Konzept einer Kalman-Filterung, insbesondere einer indirekten Kalman-Filterung, geht von der Existenz einer Stützinformation aus. Die Differenz von Informationen, die aus Messwerten von Sensoren gewonnen werden, und dieser Stützinformation dient dann als Eingangssignal des Kalman-Filters. Da das erfindungsgemäße Verfahren und die Vorrichtung aber keine kontinuierliche Information von einem Referenzsystem erhalten, steht eine Stützinformation für die Positionsbestimmung jedenfalls grundsätzlich nicht zur Verfügung. Um dennoch die Anwendung indirekter Kalman-Filterung zu ermöglichen, wird vorgeschlagen, einen zweiten, parallel angeordneten Beschleunigungssensor zu verwenden. Die Differenz der Sensorsignale der parallelen Beschleunigungssensoren dient dann als Eingangssignal für das Kalman-Filter. Figuren 3,4 und 5 zeigen schematisch das erfindungsgemäße Konzept eines redundanten Parallelsystems für die Kalman-Filterung, wobei zwei Sensoren so angeordnet sind, dass sich ihre sensitiven Sensorachsen parallel zueinander erstrecken (Figur 4).

In vorteilhafter Weise werden beide Integrationsschritte in die Modellierung aufgenommen. Man erhält also einen Schätzfehler für den durch zweifache Integration zwangsläufig sich ergebenden Positionsfehler. Figur 5 verdeutlicht dies schematisch in einer Feed-Forward-Konfiguration als konkrete Realisierung eines allgemeinen indirekten Kalman-Filters.

In diesem Konzept erzeugt ein Gauss-Markov-Prozess 1. Ordnung den Beschleunigungsfehler mit Hilfe von weißem Rauschen. Das Modell beruht darauf, dass aus dem Beschleunigungsfehler durch zweifache Integration der Positionsfehler ermittelt wird. Es ergeben sich die Gleichungen 23 und 25.

$$\dot{e}_s(t) = e_v(t) \qquad (23)$$

$$\dot{e}_v(t) = e_a(t) \qquad (24)$$

$$\dot{e}_a(t) = -\beta \cdot e_a(t) \cdot w_a(t) \qquad (25)$$

In Anlehnung an die allgemeine stochastische Zustandsraumbeschreibung eines zeitkontinuierlichen Systemmodells mit Zustandsvektor $\underline{x}(t)$, Zustandsübergangsmatrix $\underline{\Phi}(T)$, stochastischer Streumatrix $\underline{G}$ und Messrauschen $\underline{w}(t)$ ergibt sich die Systemgleichung 26 bzw. 27.

$$\dot{\underline{x}}(t) = \underline{\underline{\phi}}(T) \cdot \underline{x}(t) + \underline{G} \cdot \underline{w}(t) \qquad (26)$$

$$\begin{bmatrix} \dot{e}_s(t) \\ \dot{e}_v(t) \\ \dot{e}_a(t) \end{bmatrix} = \begin{bmatrix} 0 & 1 & 0 \\ 0 & 0 & 1 \\ 0 & 0 & -\beta \end{bmatrix} \begin{bmatrix} e_s(t) \\ e_v(t) \\ e_a(t) \end{bmatrix} + \begin{bmatrix} 0 & 0 & 0 \\ 0 & 0 & 0 \\ 0 & 0 & 1 \end{bmatrix} \begin{bmatrix} w_s(t) \\ w_v(t) \\ w_a(t) \end{bmatrix} \qquad (27)$$

Für das Messrauschen $\underline{w}(t)$ gelten die Gleichungen 28 und 29.

$$E\{\underline{w}(t)\} = 0 \qquad (28)$$

$$E\{\underline{w}(t) \cdot \underline{w}(t)^T\} = \underline{\underline{Q}}_d \cdot \delta(t - t^T) \qquad (29)$$

Die allgemeine stochastische Zustandsraumbeschreibung für das äquivalente zeitdiskrete Systemmodell ergibt sich nach Gleichung 30 bzw. 31.

$$\underline{x}(k+1 \mid k) = \underline{\underline{\phi}}(T) \cdot \underline{x}(k \mid k) + \underline{w}_d(k \mid k) \qquad (30)$$

$$\begin{bmatrix} e_s(k+1 \mid k) \\ e_v(k+1 \mid k) \\ e_a(k+1 \mid k) \end{bmatrix} = \underline{\underline{\phi}}(T) \cdot \begin{bmatrix} e_s(k \mid k) \\ e_v(k \mid k) \\ e_a(k \mid k) \end{bmatrix} + \underline{w}_d(k \mid k) \qquad (31)$$

Für die erforderliche zeitdiskrete Messgleichung gelten die Gleichungen 32 und 33.

$$y(k) = \underline{C} \cdot \underline{x}(k) + v(k) \qquad (32)$$

$$y(k) = \underline{C} \cdot \begin{bmatrix} e_s(k) \\ e_v(k) \\ e_a(k) \end{bmatrix} + v(k) \qquad (33)$$

In Gleichung 33 ist $\underline{v}(k)$ ein vektorieller weißer Rauschprozess. Als Eingang für das Kalman-Filter gilt die Differenz der beiden Sensorsignale. Damit ergeben sich für die Messgleichung die Gleichungen 34 bis 36.

$$y(k) = \Delta e_a(k) = [a_2(k) + e_{a2}(k)] - [a_1(k) + e_{a1}(k)] \qquad (34)$$

$$y(k) = e_{a2}(k) - e_{a1}(k) \qquad (35)$$

$$y(k) = \begin{bmatrix} 0 & 0 & -1 \end{bmatrix} \cdot \begin{bmatrix} e_{s1}(k) \\ e_{v1}(k) \\ e_{a1}(k) \end{bmatrix} + \begin{bmatrix} 0 \\ 0 \\ e_{a2}(k) \end{bmatrix} \qquad (36)$$

In diesem Modell sollte sowohl $\underline{e}_{a1}(k)$ als auch $\underline{e}_{a2}(k)$ als Gauss-Markov-Prozess erster Ordnung modelliert werden. Dazu dient der Ansatz nach Gleichung 37.

$$\dot{e}_{a2}(t) = -\beta \cdot e_{a2}(t) \cdot w_{a2}(t) \qquad (37)$$

Der äquivalente zeitdiskrete Ansatz dazu folgt nach Gleichung 38.

$$e_{a2}(k+1 \mid k) = e^{-\beta T} \cdot e_{a2}(k \mid k) \cdot w_{a2}(k \mid k) \qquad (38)$$

Betrachtet man $e_{a2}(k)$ als weiteren Zustand ergibt sich das erweiterte Systemmodell nach Gleichung 39 und 40.

$$\underline{x}_e(k+1 \mid k) = \underline{\underline{\phi}}_e(T) \cdot \underline{x}_e(k \mid k) + \underline{w}_{de}(k \mid k) \qquad (39)$$

$$\begin{bmatrix} e_{s1}(k+1 \mid k) \\ e_{v1}(k+1 \mid k) \\ e_{a1}(k+1 \mid k) \\ e_{a2}(k+1 \mid k) \end{bmatrix} = \underline{\underline{\phi}}_e(T) \cdot \begin{bmatrix} e_{s1}(k \mid k) \\ e_{v1}(k \mid k) \\ e_{a1}(k \mid k) \\ e_{a2}(k \mid k) \end{bmatrix} + \underline{w}_{de}(k \mid k) \qquad (40)$$

Dabei gelten die Gleichungen 41 bis 43.

$$\underline{\underline{\phi}}_e(T) = \begin{bmatrix} \underline{\underline{\phi}}(T) & 0 \\ 0 & e^{-\beta_2 \cdot T} \end{bmatrix} \qquad (41)$$

$$\underline{w}_{de} = \begin{bmatrix} \underline{w}_{a1}(k) \\ \underline{w}_{a2}(k) \end{bmatrix} \qquad (42)$$

$$\underline{Q}_{de} = \begin{bmatrix} \underline{Q}_d & 0 \\ 0 & q_{a2} \end{bmatrix} \qquad (43)$$

Für das erweiterte Messmodell gelten die Gleichungen 44 bis 47.

$$y(k) = [a_2(k) + e_{a2}(k)] - [a_1(k) + e_{a1}(k)] \qquad (44)$$

$$y(k) = e_{a2}(k) - e_{a1}(k) \qquad (45)$$

$$y(k) = \underline{C} \cdot \underline{x}(k) + v(k) \qquad (46)$$

$$y(k) = \begin{bmatrix} 0 & 0 & -1 & 1 \end{bmatrix} \cdot \begin{bmatrix} e_{s1}(k) \\ e_{v1}(k) \\ e_{a1}(k) \\ e_{a2}(k) \end{bmatrix} + v(k) \qquad (47)$$

Bei dieser Messgleichung handelt es sich um eine perfekte und damit fehlerfreie Messung, d.h. es tritt kein Messrauschen v(k) auf. Damit bedarf es einer Modellierung nach Gleichung 48.

$$R(k) = r(k) = 0 \qquad (48)$$

Die Kovarianzmatrix R des Messrauschens ist damit singulär, d.h. $R^{-1}$ existiert nicht. Die Existenz von $R^{-1}$ ist hinreichende aber nicht notwendige Bedingung für die Stabilität bzw. für die stochastische Beobachtbarkeit des Kalman-Filters. Es gibt nun zwei Möglichkeiten auf den Umstand der Singularität zu reagieren:

1. Verwendung von R=0. Das Filter kann stabil sein. Da hier ohnehin nur der Bedarf für kurzzeitige Stabilität besteht, kann auf Langzeitstabilität verzichtet werden.

2. Verwendung eines reduzierten Beobachters.

In diesem Konzept wird die varianz R=0 verwendet. Damit arbeiten die verwendeten Filter hinreichend stabil.

Die Kalman-Filter-Gleichungen für das eindimensionale System in diskreter Form ergeben sich zu Gleichungen 49 bis 50.

Bestimmung der Kalmanverstärkung nach Gleichung 49.

$$\underline{K}(k+1|) = \underline{P}(k+1|k) \cdot \underline{C}^T(k)\left[\underline{C}(k) \cdot \underline{P}(k+1|k) \cdot \underline{C}^T(k) + R(k)\right]^{-1} \qquad (49)$$

Aktualisierung der Zustandsvorhersage nach Gleichung 50.

$$\hat{\underline{x}}(k+1|k+1) = \hat{\underline{x}}(k+1|k) + \underline{K}(k+1) \cdot \tilde{y}(k+1) \qquad (50)$$

Wobei Gleichung 51 gilt.

$$\tilde{y}(k) = y(k) - \hat{y}(k) = y(k) - \underline{C}(k) \cdot \hat{\underline{x}}(k) \qquad (51)$$

Aktualisierung der Kovarianzmatrix des Schätzfehlers nach Gleichung 52 bzw. 53.

$$\underline{P}(k+1|k+1) = (\underline{I} - \underline{K}(k+1) \cdot \underline{C}^T(k)) \cdot \underline{P}(k+1|k) \cdot (\underline{I} - \underline{K}(k+1) \cdot \underline{C}^T(k))^T$$

$$+ \underline{K}(k+1) \cdot R(k) \cdot \underline{K}^T(k+1) \qquad (52)$$

$$\underline{P}(k+1|k+1) = (\underline{I} - \underline{K}(k+1) \cdot \underline{C}^T(k)) \cdot \underline{P}(k+1|k) \qquad (53)$$

Ermittlung des Prädiktionswertes des Systemzustandes nach Gleichung 54.

$$\hat{\underline{x}}(k+2|k+1) = \underline{\phi}(T) \cdot \hat{\underline{x}}(k+1|k+1) \qquad (54)$$

Ermittlung des Prädiktionswertes der Kovarianzmatrix des Schätzfehlers nach Gleichung 55.

$$\underline{P}(k+2|k+1) = \underline{\phi}(T) \cdot \underline{P}(k+1|k+1) \cdot \underline{\phi}^T(T) + \underline{Q}(k) \qquad (55)$$

Damit ist der Filterzyklus komplett durchlaufen und beginnt für den nächsten Messwert erneut. Das Filter arbeitet rekursiv, wobei die Schritte der Vorhersage und der Korrektur bei jeder Messung durchlaufen werden.

Das verwendete System beschreibt eine dreidimensionale Translation in drei orthogonalen Raumachsen. Diese Translationen werden durch den Weg s, die Geschwindigkeit v und die Beschleunigung a beschrieben. Für ein indirektes Kalman-Filter liefert jeweils ein zusätzlicher Beschleunigungssensor für jede Raumrichtung ebenfalls eine Beschleunigungsinformation.

Der grundsätzliche Algorithmus des Aufbaus wird in Bild 5 dargestellt. Das eigentliche Messsignal kommt für jede Raumachse von einem Beschleunigungssensor in Form der Beschleunigung a. Mit Hilfe der Zusatzinformation in Form des Sensorsignals des zweiten Beschleunigungssensors für jede Raumachse liefert der Kalman-Filter-Algorithmus einen Schätzwert für die Abweichung des Beschleunigungssignals ea für die drei Raumrichtungen x, y und z.

Weitere Merkmale, Einzelheiten und Vorteile der Erfindung ergeben sich aus den Patentansprüchen und aus der zeichnerischen Darstellung und nachfolgenden Beschreibung der Erfindung. In der Zeichnung zeigt:

Figur 1     die Darstellung der Rotation eines Vektors mittels Quaternionen;

Figur 2     ein Flussdiagramm, welches die Anwendung des Quaternionen-Algorithmus verdeutlicht;

Figur 3     ein Flussdiagramm, welches die Durchführung des erfindungsgemäßen Verfahrens verdeutlicht;

Figur 4     die schematische Darstellung eines Beschleunigungssensors und eines parallel hierzu angeordneten redundanten Beschleunigungssensors;

Figur 5     die schematische Andeutung eines Kalman-Filters mit INS-Fehlermodellierung in Feed-Forward-Konfiguration;

Figur 6     eine schematische Darstellung des Ergebnisses der Anwendung einer Kalman-Filterung.

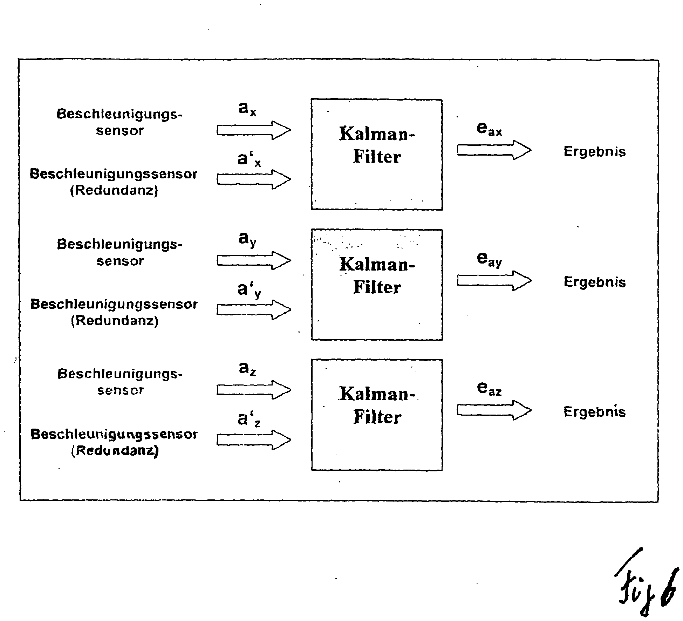

Die Figuren 1, 2 und 4 bis 6 wurden bereits vorausgehend erläutert.

Wie bereits erwähnt, wird zu Beginn der Objektverfolgung der zu positionierende Gegenstand an vorbestimmter Stelle mit der ersten Sensoreinrichtung versehen. Der Gegenstand wird dann im Raum zur Ruhe gebracht und derart mit einem ortsfesten Koordinatensystem, beispielsweise der OP-Tisch, referenziert, dass die aus den Signalen der Drehratensensoren und Beschleunigungssensoren ermittelten Winkel und Beschleunigungen zunächst zu 0 gesetzt werden. Es wird dann während einer absoluten Ruhephase des Gegenstands ein Offset-Wert ermittelt, der bei jeder Abtastung, d. h. bei jeder Datennahme, berücksichtigt wird. Es handelt sich hierbei um einen Driftvektor, dessen Komponenten die ermittelten Offset-Werte der Sensoren umfassen.

Als ganz besonders vorteilhaft erweist es sich, dass jedes Mal, wenn ein Ruhen der Sensoreinrichtung detektiert und zu Grunde gelegt wird, die Offset-Werte der Sensoren von neuem ermittelt und der weiteren Berechnung der Position und Orientierung zugrundegelegt werden.

Ferner wird die zuvor erwähnte Ausgleichsmatrix ermittelt, die einer Abweichung der Achsen der Drehratensensoren von einer angenommenen Orientierung zueinander und zu einem Gehäuse der Sensoreinrichtung entspricht bzw. diese Abweichung genau ausgleichen soll.

Die vorstehenden Ausführungen gelten für die zweite Sensoreinrichtung, die am Patienten festgelegt werden soll, entsprechend.

Bei der Durchführung des Positionierens und Orientierens werden zu aufeinander folgenden Zeitpunkten, etwa mit einer Abtastrate von 10 bis 30 Hz, insbesondere von ca. 20 Hz, die Sensorsignale erfasst und durch einfache oder zweifache Integration in infinitesimale Drehwinkel bzw. in Positionsdaten umgerechnet. Dabei wird aber die Ausgleichsmatrix für die Nichtorthogonalität der Drehratensensoren berücksichtigt, um zu einer erhöhten Genauigkeit bei der Ermittlung der Orientierung zu gelangen.

Anhand der infinitesimal kleinen Winkeländerungen, die jeweils aus den Messsignalen der drei Drehratensensoren ermittelt wurden, könnte nun nach dem Eulerschen Verfahren durch Angabe von drei Winkeln die Orientierung des verdrehten Koordinatensystems des Objekts zum Referenzkoordinatensystem ermittelt werden. Stattdessen erweist es sich als vorteilhaft, wenn zur Ermittlung der Orientierung ein Quaternionen-Algorithmus der eingangs beschriebenen Art durchgeführt wird. Hierdurch kann anstelle von drei nacheinander auszuführenden Drehungen eine einzige Transformation angenommen werden, was die Genauigkeit der hieraus gewonnenen Orientierung des Objektsystems weiter erhöht.

Im Ergebnis der Durchführung des Quaternionen-Algorithmus ist die Orientierung des Objekts im Raum gegeben.

Wie in Figur 3 angedeutet, ist es jedoch möglich, durch weitere Sensoren, etwa eine dreidimensionale Magnetfeldsensorik, zu beliebigen Zeitpunkten das auf das Objekt einwirkende Magnetfeld zu bestimmen, wobei es sich hierbei um das an einem Ort bekannte Erdmagnetfeld handelt. Des weiteren ist es denkbar, zusätzlich eine dreidimensionale Beschleunigungssensorik zur Messung der Erdbeschleunigung vorzusehen. Die Messsignale der Magnetfeldsensoren und der Beschleunigungssensoren lassen sich zu einem elektronischen dreidimensionalen Kompass kombinieren, der mit hoher Genauigkeit die Orientierung des Objekts im Raum angeben kann, wenn keine Störeffekte vorhanden sind, vorzugsweise wenn die Messwerte während eines Ruhens des Objekts genommen wurden. Die hieraus gewonnene Orientierung des Objekts im Raum lässt sich als Stützinformation für diejenige Orientierung, die nur durch die Signale der drei Drehratensensoren gewonnen wurde, verwenden. Zunächst werden die Messsignale der Magnetfeldsensoren und der Beschleunigungssensoren daraufhin untersucht, ob sie von Störungen beeinflusst sind oder nicht.

Wenn dies nicht der Fall ist, so werden sie als bei der Ausführung des Verfahrens zu berücksichtigende Stützinformation mit der Orientierungsinformation, die aus den drei Drehratensensoren gewonnen wurde, verglichen. Hierfür wird in vorteilhafter Weise ein Kalman-Filter-Algorithmus verwendet. Hierbei handelt es sich um einen Schätzalgorithmus, bei dem die aus dem vorausgehend erwähnten dreidimensionalen Kompass ermittelten Informationen über die Orientierung des Objekts als korrekte Stützinformation zugrundegelegt werden, wenn sie mit denjenigen Informationen über die Orientierung verglichen werden, die aus den Drehratensensoren gewonnen wurden.

Wie vorausgehend im Einzelnen beschrieben wurde, können auch die Messwerte der Beschleunigungssensoren durch Anwendung einer Kalman-Filterung verbessert werden, indem nämlich als Ersatz für eine anderweitig zugängliche Stützinformation vorzugsweise für jeden Beschleunigungssensor ein parallel hierzu angeordneter redundanter Beschleunigungssensor vorgesehen wird. Mit Hilfe dieser Zusatzinformation in Form des Messwertsignals des zweiten Beschleunigungssensors für jede Raumachse kann ein Schätzwert für die fehlerbedingte Abweichung des Beschleunigungsmesswertsignals für die jeweilige Raumrichtung ermittelt werden.

**Patentansprüche**

1. Verfahren zum Navigieren und Positionieren eines Gegenstands relativ zu einem Patienten während einer medizinischen Operation im Operationssaal, **dadurch gekennzeichnet, dass** mittels einer dreidimensionalen Inertialsensorik die Position und die Orientierung im Raum sowohl des Gegenstands als auch des Patienten oder eines betreffenden Bereichs des Patienten relativ zu einem referenzierenden Bezugssystem quasikontinuierlich entsprechend einer Abtastrate ermittelt wird, und dass hieraus die momentane Position und Orientierung des Gegenstand relativ zu dem Patienten ermittelt wird, dass diese Position und Orientierung mit einer erwünschten vorbestimmten Position und Orientierung verglichen wird und dass angezeigt wird, in welcher Weise der Gegenstand lageverändert werden muss, um in die erwünschte vorbestimmte Position und Orientierung zu gelangen.

2. Verfahren nach Anspruch 1, **dadurch gekennzeichnet, dass** die Abtastrate 10 - 50 Hz, insbesondere 10 - 40 Hz, insbesondere 10 - 30 Hz und weiter insbesondere 15 - 25 Hz beträgt.

3. Vorrichtung zur Durchführung des Verfahrens nach Anspruch 1 oder 2, umfassend eine erste an vorbestimmter Stelle des Gegenstands festlegbare und wieder lösbare Sensoreinrichtung mit Beschleunigungssensoren und Drehratensensoren und eine zweite an dem Patienten befestigbare und wieder lösbare Sensoreinrichtung mit Beschleunigungssensoren und Drehratensensoren, eine Speichereinrichtung, in der die erwünschte vorbestimmte Position und Orientierung des Gegenstands relativ zu dem Patienten abgelegt ist, und Rechenmittel zum Bestimmen der Position und Orientierung aus den Sensormesswerten und Rechenmittel zum Vergleichen der ermittelten Position und Orientierung mit der vorbestimmten Position und Orientierung und Anzeigemittel zum Anzeigen, in welcher Weise der Gegenstand lageverändert werden muss, um in die erwünschte vorbestimmte Position und Orientierung zu gelangen.

4. Vorrichtung nach Anspruch 3, **dadurch gekennzeichnet, dass** mit einer Abtastrate von 10 - 50 Hz, insbesondere von 10 - 40 Hz, insbesondere von 10 - 30 Hz und weiter insbesondere von 15 - 25 Hz die Messwerte der Sensoreinrichtung erfassbar und weiterverarbeitbar sind.

5. Vorrichtung nach Anspruch 3 oder 4, **dadurch gekennzeichnet, dass** die Sensoreinrichtungen eine Orientierungshilfe aufweisen, die eine korrekte Festlegung an dem Gegenstand bzw. an dem Patienten gestattet.

6. Vorrichtung nach Anspruch 3, 4 oder 5, **dadurch gekennzeichnet, dass** die Sensoreinrichtungen Befestigungsmittel aufweisen zum lösbare Festlegen der Sensoreinrichtung an dem Gegenstand und an dem Patienten.

7. Vorrichtung nach einem oder mehreren der Ansprüche 3-6, **dadurch gekennzeichnet, dass** die erste und insbesondere auch die zweite Sensoreinrichtung drei Beschleunigungssensoren, deren Signale zur Berechnung einer translatorischen Bewegung verwendbar sind, und zusätzlich drei Drehratensensoren, deren Messwerte zum Bestimmen der Orientierung im Raum verwendbar sind, umfasst.

8. Vorrichtung nach einem oder mehreren der Ansprüche 3-7, **dadurch gekennzeichnet, dass** die Rechenmittel Mittel zur Ausführung eines Quaternionen-Algorithmus aufweisen.

9. Vorrichtung nach einem oder mehreren der Ansprüche 3-8, **dadurch gekennzeichnet, dass** die Rechenmittel Mittel zur Anwendung einer vor Beginn der Positionierung ermittelten und gespeicherten Ausgleichsmatrix aufweisen, welche Ausgleichsmatrix einer Abweichung der Orientierung der Achsen der drei Drehratensensoren von einer angenommenen Orientierung der Achsen zueinander Rechnung trägt und durch ihre Anwendung einen bei der Berechnung der Drehwinkel resultierenden Fehler kompensiert.

**10.** Vorrichtung nach einem oder mehreren der Ansprüche 3-9, **dadurch gekennzeichnet, dass** die Rechenmittel Mittel zur Ausführung eines Kalman-Filter-Algorithmus aufweisen.

**11.** Vorrichtung nach einem oder mehreren der Ansprüche 3-10, **dadurch gekennzeichnet, dass** zusätzlich zu den Drehratensensoren Magnetfeldsensoren zur Ermittlung der Orientierung des Gegenstands im Raum vorgesehen sind.

**12.** Vorrichtung nach Anspruch 11, **dadurch gekennzeichnet, dass** Mittel zum Vergleichen der aus Messwerten der Magnetfeldsensoren ermittelten Orientierung im Raum mit der aus Messwerten der Drehratensensoren ermittelten Orientierung im Raum vorgesehen sind.

**13.** Vorrichtung nach Anspruch 11 oder 12, **dadurch gekennzeichnet, dass** Mittel zum Vergleichen der aus Messwerten der Magnetfeldsensoren ermittelten Orientierung im Raum mit der aus Messwerten eines Beschleunigungssensors für die Messung der Erdbeschleunigung ermittelten Orientierung im Raum vorgesehen sind.

**14.** Vorrichtung nach einem oder mehreren der Ansprüche 3-13, **dadurch gekennzeichnet, dass** zur Durchführung einer Kalman-Filterung für jeden Beschleunigungssensor ein parallel hierzu angeordneter redundanter Beschleunigungssensor vorgesehen ist.

## Claims

**1.** Method for navigating and positioning an object relative to a patient during surgery in an operating theatre, **characterized in that** the position and orientation in the room space of both the object and the patient or of a relevant area of the patient relative to a referencing framework is determined quasi continuously according to a sensing rate by means of three-dimensional inertial sensors, and that from this the current position and orientation of the object relative to the patient is determined, that this position and orientation are compared with a desired, predetermined position and orientation, and that there is an indication as to how the position of the object should be modified in order to be placed in the desired predetermined position and orientation.

**2.** Method according to Claim 1, **characterized in that** the sensing rate is 10 - 50 Hz, especially 10 - 40 Hz, especially 10 - 30 Hz and further especially 15 - 25 Hz.

**3.** Apparatus for the implementation of the method according to Claim 1 or 2 comprising a first sensor device with acceleration sensors and rotational rate sensors that is attachable to and again removable from a first predetermined area of the object, and a second sensor device with acceleration sensors and rotational rate sensors that is attachable to and again removable from a patient, a memory, where the desired predetermined position and orientation of the object relative to the patient is stored, and computing means to determine the position and orientation from the measured sensor values, and computing means to compare the determined position and orientation with the predetermined position and orientation, and indication means to indicate how the position of the object should be modified in order to be placed into the desired predetermined position and orientation.

**4.** Apparatus according to Claim 3, **characterized in that** the measured values of the sensor device can be acquired and processed at a sensing rate of 10 - 50 Hz, especially 10 - 40 Hz, especially 10 - 30 Hz and further especially 15 - 25 Hz.

**5.** Apparatus according to Claim 3 or 4, **characterized in that** the sensor devices have an orientation aid, which allows correct fastening to the object and to the patient, respectively.

**6.** Apparatus according to Claim 3, 4 or 5, **characterized in that** the sensor devices have means of fastening for the removable attachment of the sensor device to the object and the patient.

**7.** Apparatus according to one or more of Claims 3 - 6, **characterized in that** the first and, especially also the second sensor device comprise three acceleration sensors, whose signals may be used for the calculation of translational movements, and in addition three rotational rate sensors, whose measured values may be used for the determination of the orientation in the room.

8. Apparatus according to one or more of Claims 3 - 7, **characterized in that** the computing means comprise means for the execution of a quaternion algorithm.

9. Apparatus according to one or more of Claims 3 - 8, **characterized in that** the computing means comprise means for the application of a compensation matrix that is determined and stored prior to the start of positioning, said compensation matrix allowing for a deviation of the axial orientation of the three rotational rate sensors from an assumed orientation of the axes toward each other and compensating for errors resulting from the calculation of the rotation angles.

10. Apparatus according to one or more of Claims 3 - 9, **characterized in that** the computing means have means for executing a Kalman filter algorithm.

11. Apparatus according to one or more of claims 3 - 10, **characterized in that** in addition to the rotational rate sensors, magnetic field sensors for the determination of the space orientation of the object are provided.

12. Apparatus according to Claim 11, **characterized in that** means for comparing the space orientation determined from the values measured by the magnetic field sensors with the space orientation determined by the values measured by the rotational rate sensors are provided.

13. Apparatus according to Claim 11 or 12, **characterized in that** means for comparing the space orientation determined from the values measured by the magnetic field sensors with the space orientation determined by the values measured by a gravitational acceleration sensor are provided.

14. Apparatus according to one or more of Claims 3 - 13, **characterized in that** for each acceleration sensor a redundant acceleration sensor arranged parallel to it is provided for the implementation of Kalman filtering.

**Revendications**

1. Procédé pour faire circuler et positionner un objet par rapport à un patient pendant une opération médicale en salle d'opération, **caractérisé en ce que** la position et l'orientation dans l'espace aussi bien de l'objet que du patient ou d'une région considérée du patient par rapport à un référentiel de référence sont déterminées au moyen d'une sensorique inertielle tridimensionnelle de façon quasi continue, avec un certaine cadence de scrutation, et **en ce que** la position et l'orientation instantanées de l'objet par rapport au patient sont déterminées sur cette base, que cette position et cette orientation sont comparées à une position et une orientation prédéterminées souhaitées et que la façon dont l'objet doit être déplacé pour atteindre la position et l'orientation prédéterminées souhaitées est indiquée.

2. Procédé selon la revendication 1, **caractérisé en ce que** la cadence de scrutation est de 10 à 50 Hz, en particulier 10 à 40 Hz, et plus particulièrement de 15 à 25 Hz

3. Dispositif pour la mise en oeuvre du procédé selon la revendication 1 ou 2, comprenant un premier dispositif capteur comportant des capteurs d'accélération et des capteurs de vitesse de rotation et qui peut être mis en place à un endroit prédéterminé de l'objet et en être de nouveau éloigné, et un second dispositif capteur comprenant des capteurs d'accélération et des capteurs de vitesse de rotation et qui peut être fixé au patient et en être de nouveau détaché, un dispositif de mémoire dans lequel la position et l'orientation prédéterminées souhaitées de l'objet par rapport au patient sont mémorisées, des moyens de calcul servant à déterminer la position et l'orientation à partir des valeurs mesurées de capteurs, des moyens de calcul servant à comparer la position et l'orientation déterminées à la position et à l'orientation prédéterminées et des moyens indicateurs servant à indiquer la façon dont l'objet doit être déplacé pour atteindre la position et l'orientation prédéterminées souhaitées.

4. Dispositif selon la revendication 3, **caractérisée en ce que** les valeurs mesurées du dispositif capteur peuvent être acquises et traitées avec une cadence de scrutation de 10 à 50 Hz, en particulier de 10 à 40 Hz, plus particulièrement de 10 à 30 Hz et encore plus particulièrement de 15 à 25 Hz.

**5.** Dispositif selon la revendication 3 ou 4, **caractérisé en ce que** les dispositifs capteurs présentent une aide à l'orientation qui permet la mise en place correcte sur l'objet ou sur le patient.

**6.** Dispositif selon la revendication 3, 4 ou 5, **caractérisé en ce que** le dispositif capteur présente des moyens de fixation pour la fixation démontable du dispositif capteur sur l'objet et sur le patient.

**7.** Dispositif selon une ou plusieurs des revendications 3 à 6, **caractérisé en ce que** le premier dispositif capteur et en particulier aussi le deuxième dispositif capteur peut ou peuvent comprendre trois capteurs d'accélération dont les signaux peuvent être utilisés pour le calcul d'un mouvement de translation et, en supplément, trois capteurs de vitesse de rotation dont les valeurs mesurées peuvent être utilisées pour la détermination de l'orientation dans l'espace.

**8.** Dispositif selon une ou plusieurs des revendications 3 à 7, **caractérisé en ce que** les moyens de calcul présentent des moyens pour l'exécution d'un algorithme de quaternions.

**9.** Dispositif selon une ou plusieurs des revendications 3 à 8, **caractérisé en ce que** les moyens de calcul présentent des moyens pour l'utilisation d'une matrice de compensation déterminée et mémorisée, laquelle matrice de compensation tient compte d'une déviation de l'orientation des axes des trois capteurs de vitesse de rotation par rapport à une orientation supposée des axes les uns par rapport aux autres et compense par son application une erreur qui se produit dans le calcul des angles de rotation

**10.** Dispositif selon une ou plusieurs des revendications 3-9, **caractérisé en ce que** les moyens de calcul présentent des moyens pour l'exécution d'un algorithme à filtre de Kalmann.

**11.** Dispositif selon une ou plusieurs des revendications 3-10, **caractérisé en ce que** des capteurs de champ magnétique servant à déterminer l'orientation de l'objet dans l'espace sont prévus en supplément des capteurs de vitesse de rotation.

**12.** Dispositif selon la revendication 11, **caractérisée en ce qu'**il est prévu des moyens pour comparer l'orientation dans l'espace déterminée à partir des valeurs mesurées des capteurs de champ magnétique à l'orientation dans l'espace déterminée à partir des valeurs mesurées des capteurs de vitesse de rotation.

**13.** Dispositif selon la revendication 11 ou 12, **caractérisé en ce qu'**il est prévu des moyens servant à comparer l'orientation dans l'espace déterminée à partir des valeurs mesurées des capteurs de champ magnétique à l'orientation dans l'espace déterminée à partir des valeurs mesurées des capteurs d'accélération servant à la mesure de l'accélération terrestre.

**14.** Dispositif selon une ou plusieurs des revendications 3-13, **caractérisé en ce que**, pour l'exécution d'un filtrage de Kalman pour chaque capteur d'accélération, il est prévu un capteur d'accélération redondant disposé en parallèle avec chaque capteur d'accélération.

*Fig 1*

*Fig 2*

*fig 3*

$a_{x2}$ □ ‖ □ $a_{x1}$

*Fig 4*

$a_1(k) + e_{a1}(k)$
**Beschleunigungssensor 1**

$v_1(k) + e_{v1}(k)$

$s_1(k) + e_{s1}(k)$

$s(k)$

$e_s(k)$

$a_2(k) + e_{a2}(k)$
**Beschleunigungssensor 2**

$\Delta e_a(k)$

**Kalman
Filter**

*Fig 5*

Beschleunigungssensor $\quad a_x$

Beschleunigungssensor $\quad a'_x$
(Redundanz)

Kalman-
Filter $\quad e_{ax}$     Ergebnis

Beschleunigungssensor $\quad a_y$

Beschleunigungssensor $\quad a'_y$
(Redundanz)

Kalman-
Filter $\quad e_{ay}$     Ergebnis

Beschleunigungssensor $\quad a_z$

Beschleunigungssensor $\quad a'_z$
(Redundanz)

Kalman-
Filter $\quad e_{az}$     Ergebnis

Fig 6

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 4225112
- DE 10312154 A1